# EUROPÄISCHE PATENTSCHRIFT

(11) **EP 3 513 752 B1**
(45) Veröffentlichungstag und Bekanntmachung des Hinweises auf die Patenterteilung: **14.12.2022**
(21) Anmeldenummer: 19152363.8
(22) Anmeldetag: 17.01.2019
(51) Int. Cl.: A61B 17/34, A61B 5/00

(54) **INJEKTOR ZUM TRANSKUTANEN EINFÜHREN EINES SENSORS IN EINEN PATIENTEN**
INJECTOR FOR TRANSCUTANEOUS INSERTION OF A SENSOR IN A PATIENT
INJECTEUR DESTINÉ À L'INTRODUCTION TRANSCUTANE D'UN CAPTEUR DANS UN PATIENT

(30) Priorität: 22.01.2018 DE 102018101275
(43) Veröffentlichungstag der Anmeldung: 24.07.2019
(73) Patentinhaber: EyeSense GmbH, 63762 Großostheim (DE)
(72) Erfinder: Müller, Achim, 63762 Großostheim (DE); Meissner-Braun, Tom, 88662 Überlingen (DE); Pischan, Matthias, 64287 Darmstadt (DE)
(74) Vertreter: LBP Lemcke, Brommer & Partner Patentanwälte mbB

(56) Entgegenhaltungen:
- EP-A1- 2 250 959
- US-A- 5 390 671
- US-A1- 2016 310 051
- US-A1- 2017 303 831
- US-A9- 2016 008 028

## Beschreibung

Die Erfindung betrifft einen Injektor zum transkutanen Einführen eines Sensors in einen Patienten gemäß Oberbegriff des Anspruchs 1.

Für vielfältige medizinische Anwendungen ist das Einführen eines Sensors in einen Patienten notwendig, insbesondere, um Messwerte des Patienten zu erhalten, wie beispielsweise Glukosewerte oder Laktosewerte.

Aus US 2016/0008028 A9 ist eine Injektionsvorrichtung bekannt, mit der ein Sensor mittels einer geschlitzten Kanüle in das Gewebe eines Patienten injiziert werden kann. Ein Halteelement ist mit dem Sensor verbunden und erlaubt die Einstellung seiner relativen Position zu der Kanüle.

Aus US 2017/0303831 A1 ist ebenso eine Injektionsvorrichtung bekannt, bei der zur Durchführung einer Injektion ein Injektionsaufsatz an einer Basisplatte angeordnet wird. Die Basisplatte selbst ist dabei an einem Patienten angeordnet und umfasst Klemmelemente, die ein ungewolltes Herausziehen des Sensors nach dem Injektionsvorgang verhindern.

Aus US 2016/0310051 A1 ist eine andere Injektionsvorrichtung bekannt, mit der ein Sensor über eine Kanüle in ein Patientengewebe eingeführt werden kann. Ein Kolbenelement erlaubt es, den Sensor im Patienten zu belassen, wenn die Kanüle selbst herausgezogen wird.

Aus US 2004/0133164 A1 ist ein Injektor zum transkutanen Einführen eines Sensors in einen Patienten bekannt, bei welchem eine geschlitzte Kanüle und ein sich darin befindender Sensor mittels eines Schiebeelementes transkutan in einen Patienten eingeführt werden. Die Kanüle weist hierzu durchgängig einen Schlitz in Längsrichtung der Kanüle auf. Der Sensor wird beim Herausziehen der Kanüle durch die Reibung am Gewebe des Patienten gehalten, insbesondere durch einen Wiederhaken, um nicht herausgezogen zu werden.

Es sind jedoch mehr Freiheiten hinsichtlich der Geometrie des Sensors und weniger Anforderungen hinsichtlich der Reibungseigenschaften zwischen Sensor und Gewebe wünschenswert.

Der vorliegenden Erfindung liegt die Aufgabe zugrunde, einen Injektor zum transkutanen Einführen eines Sensors in einen Patienten zu schaffen, der geringere Anforderungen an die Rückhaltekraft des Sensors im Gewebe des Patienten beim Herausziehen der Kanüle stellt.

Gelöst ist diese Aufgabe durch einen Injektor gemäß Anspruch 1. Vorteilhafte Ausgestaltungen des erfindungsgemäßen Injektors finden sich in den abhängigen Unteransprüchen.

I

Der erfindungsgemäße Injektor zum transkutanen Einführen eines Sensors in einen Patienten weist eine Kanüle, einen in der Kanüle angeordneten Sensor, ein Basiselement, und ein in einer Injektionsrichtung verschiebbar an dem Basiselement angeordnetes Schiebeelement zum transkutanen Einführen der Kanüle mit dem Sensor in den Patienten in einem Injektionsvorgang auf. Bei dem Injektionsvorgang werden somit durch Verschieben des Schiebeelementes in einer Injektionsrichtung (in Richtung des Patienten) Kanüle und Sensor transkutan in den Patienten eingeführt. Die Kanüle weist zumindest in einem proximalen Bereich einen Schlitz in einer Längsrichtung der Kanüle auf.

Wesentlich ist, dass der Injektor ein verschiebbar an dem Basiselement angeordnetes Halteelement aufweist, um in einem Ejektionsvorgang bei einem Herausziehen der Kanüle aus dem Patienten ein Herausziehen des Sensors mittels des Halteelementes zu verhindern. Das Halteelement greift im Bereich eines distalen Endes des Sensors durch den Schlitz in die Kanüle ein.

Halteelement und/oder Basiselement weisen zumindest ein Festlegemittel auf, so dass im Zustand der in den Patienten transkutan eingeführten Kanüle das Halteelement mittelbar oder bevorzugt unmittelbar an dem Basiselement selbsttätig arretierbar ist.

Bei dem Injektionsvorgang werden somit Kanüle, Sensor und Halteelement mittels des Schiebeelementes aus einer distalen Startposition zu einer proximalen Endposition relativ zu dem Basiselement verschoben, so dass bei dem Injektionsvorgang in Injektionsrichtung Sensor und Kanüle transkutan in den Patienten eingeführt werden.

Da das Halteelement im Bereich eines distalen Endes des Sensors durch den Schlitz in die Kanüle eingreift und zum selbsttätigen Arretieren mittelbar oder bevorzugt unmittelbar an dem Basiselement ausgebildet ist, erfolgt in der Endstellung des Injektionsvorgangs das Arretieren des Halteelementes, so dass bei Herausziehen der Kanüle entgegen der Injektionsrichtung in einem Ejektionsvorgang das Halteelement diese Bewegung nicht vollzieht und durch das Eingreifen in den Schlitz der Kanüle im Bereich des distalen Endes des Sensors, insbesondere oberhalb des distalen Endes des Sensors, ebenso eine Bewegung des Sensors und somit ein Herausziehen des Sensors verhindert. Der Sensor verbleibt somit auch beim Ejektionsvorgang in der Injektionsposition, insbesondere einer transkutanen Injektionsposition, unabhängig von einer etwaigen Reibung oder Haftung zwischen Sensor und Gewebe des Patienten.

Erfindungsgemäß wird der Injektor nach Injektion des Sensors entfernt, um ein Detektionselement an dem Patienten mit dem Sensor zu verbinden und Daten aufzunehmen und bevorzugt drahtlos an eine Auswerteeinheit weiterzuleiten.

Daher sind Halteelement und Sensor trennbar ausgebildet, so dass bei Entfernen des Injektors auch das Halteelement entfernt werden kann, ohne den Sensor herauszuziehen. Insbesondere ist es daher vorteilhaft, Halteelement und Sensor als separate Einheiten auszubilden.

In einer vorteilhaften Ausführungsform ist das Festlegemittel als Rastelement ausgebildet und eines der beiden Elemente Halteelement und Basiselement weist das Rastelement auf und das andere der beiden Elemente weist eine korrespondierende Vertiefung und/oder Ausnehmung für das Rastelement auf. Insbesondere weist das Festlegemittel bevorzugt zumindest eine Rastnase auf, welche am Ende des Injektionsvorgangs in eine entsprechende Vertiefung und/oder Ausnehmung eingreift und das Halteelement hierdurch selbsttätig an dem Basiselement arretiert. Ebenso kann umgekehrt das Basiselement die Rastnase aufweisen, welche am Ende des Injektionsvorgangs in eine entsprechende Vertiefung und/oder Ausnehmung des Halteelementes eingreift.

Bevorzugt ist die Kanüle mit ihrem distalen Ende an einem verschiebbar an dem Basiselement angeordneten Kanülenoberteil angeordnet und das Schiebeelement und Kanülenoberteil sind derart zusammenwirkend ausgebildet, dass bei dem Injektionsvorgang das Kanülenoberteil mittels des Schiebeelementes in eine Injektionsrichtung verschiebbar ist. Die Krafteinwirkung auf die Kanüle erfolgt in dieser bevorzugten Ausführungsform somit mittelbar, indem der Benutzer das Schiebeelement in Richtung des Patienten verschiebt und hierdurch eine Krafteinwirkung über das Kanülenoberteil auf die Kanüle erfolgt, um die Kanüle transkutan in den Patienten einzubringen.

Vorteilhafterweise ist hierbei das Halteelement derart mit dem Kanülenoberteil zusammenwirkend ausgebildet, dass bei dem Injektionsvorgang das Halteelement mittels des Kanülenoberteils in Injektionsrichtung verschiebbar ist.

Hierdurch wird eine baulich einfache Ausgestaltung ermöglicht, so dass bei dem Injektionsvorgang eine Krafteinwirkung des Schiebeelementes über das Kanülenoberteil auf das Halteelement erfolgt. Bevorzugt ist das Halteelement auf der dem Patienten zugewandten Seite des Kanülenoberteils angeordnet.

Insbesondere ist es vorteilhaft, dass das Halteelement die Kanüle umschließend ausgebildet ist, um eine konstruktiv einfache Ausgestaltung zu erzielen.

Die Kanüle ist bevorzugt fest, insbesondere unlösbar mit dem Oberteil verbunden. Vorteilhafterweise umschließt das Kanülenoberteil die Kanüle an einem distalen Ende der Kanüle.

Vorteilhafterweise weist das Kanülenoberteil ein Zentralelement und mindestens einen Führungsfortsatz auf, insbesondere einen Führungszapfen. Das Basiselement weist bevorzugt mindestens eine Führungswand mit einem Schlitz für den Führungsfortsatz des Kanülenoberteils auf, um das Kanülenoberteil in Injektionsrichtung zu führen. Die Kanüle ist bei dieser vorteilhaften Ausgestaltung an dem Zentralelement angeordnet. Vorteilhafterweise greift das Schiebeelement an den Führungsfortsatz an, so dass die Kraftübertragung bei Betätigen des Schiebeelementes über den Führungsfortsatz auf das Zentralelement auf die Kanüle erfolgt. Insbesondere ist es vorteilhaft, dass der Führungsfortsatz die Führungswand des Basiselementes durchdringt und das Schiebeelement an der dem Zentralelement des Kanülenoberteils abgewandten Seite der Führungswand an dem Führungsfortsatz angreifend ausgebildet ist. Hierdurch ist eine effektive Führung des Kanülenoberteils in Injektionsrichtung durch die Führungswand und den Führungsschlitz in der Führungswand gegeben.

In einer vorteilhaften Weiterbildung wird ein Verkippen des Kanülenoberteils bei Verschieben in Injektionsrichtung vermieden, indem an zwei gegenüberliegenden Seiten jeweils ein Führungsfortsatz an dem Kanülenoberteil ausgebildet ist und das Basiselement korrespondierend zumindest zwei Führungsschlitze in Injektionsrichtung für die beiden Führungsfortsätze aufweist und das Schiebeelement an beiden Führungsfortsätzen angreifend ausgebildet ist.

Die beiden Führungsschlitze können hierbei in einer gemeinsamen Führungswand des Basiselementes ausgebildet sein. Insbesondere ist es vorteilhaft, dass die Basiswand das Zentralelement des Kanülenoberteils und bevorzugt auch das Halteelement umschließend ausgebildet ist. Insbesondere ist eine Führungswand in Form eines Hohlzylinders für einen stabilen Aufbau vorteilhaft. Ebenso liegt es im Rahmen der Erfindung, dass das Basiselement mehrere Führungswände für das Kanülenoberteil und bevorzugt für das Halteelement aufweist.

In einer vorteilhaften Ausgestaltung weist der Injektor ein Ejektionselement, insbesondere eine Ejektionsfeder und ein Arretierungselement, insbesondere ein Ejektionsfederhalteelement auf. Das Ejektionsfederhalteelement ist an dem Basiselement festlegbar ausgebildet, so dass die Ejektionsfeder mittels des Ejektionsfederhalteelements in einem gespannten oder komprimierten Zustand festlegbar ist.

Hierdurch kann herstellerseitig die Ejektionsfeder z.B. in einem komprimierten Zustand angeordnet werden, so dass die hierdurch gespeicherte Energie zur Ejektion der Kanüle verwendet werden kann und somit eine automatische Ejektion erfolgt.

Vorteilhafterweise ist hierbei das Schiebeelement mit dem Arretierungselement zusammenwirkend ausgebildet, so dass mittels des Schiebeelements das Arretierungselement am Ende des Injektionsvorgangs aus einer Festlegestellung lösbar ist, insbesondere bevorzugt durch Drehung des Arretierungselement mittels des Schiebeelements, bevorzugt eine Drehung um eine Injektionsachse.

Die Injektionsachse entspricht der Achse, entlang derer die Kanüle und der Sensor bei dem Injektionsvorgang in Injektionsrichtung verschoben werden und entlang derer die Kanüle bei dem Ejektionsvorgang entgegen der Injektionsrichtung verschoben wird. Die Injektionsachse verläuft bevorzugt durch eine Mittelachse der Kanüle.

Vorteilhafterweise sind das Schiebelement und das Arretierungselement um eine gemeinsame Injektionsachse drehbar an dem Basiselement angeordnet. Hierdurch ergibt sich ein konstruktiv einfacher Aufbau.

Vorteilhafterweise weisen Basiselement und/oder Schiebeelement zumindest eine Schräge auf, insbesondere eine Kulisse, die derart angeordnet ist, dass bei dem Injektionsvorgang in einem proximalen Endbereich bei Verschieben des Schiebeelements in Injektionsrichtung eine Drehung des Schiebeelements relativ zu dem Basiselement erfolgt. Hierdurch wird in konstruktiv einfacher Weise bei vollständig oder nahezu vollständig eingeführter Kanüle eine Drehung des Schiebeelementes erzielt, um das Arretierungselement zu lösen und eine Expansion bzw. Kontraktion der Ejektionsfeder mit Ejektion der Kanüle einzuleiten.

Vorteilhafterweise weisen daher Schiebelement und Arretierungselement korrespondierende Anlageflächen auf, die derart angeordnet sind, dass durch Drehung des Schiebelements das Ejektionselement aus einer Festlegestellung lösbar ist. Bevorzugt weist das Arretierungselement einen Fortsatz und das Schiebeelement eine korrespondierende Führungsfläche auf.

Vorteilhafterweise weisen Basiselement und Schiebeelement korrespondierende Führungselemente auf, die derart ausgebildet und angeordnet sind, dass nur in dem proximalen Endbereich das Schiebeelement relativ zu dem Basiselement drehbar ist.

Hierdurch wird vermieden, dass der Benutzer vor dem proximalen Endbereich bereits eine Drehung des Schiebeelementes relativ zu dem Basiselement durchführt, die zu einer Fehlfunktion insbesondere zu einem vorzeitigen oder ausbleibenden Auslösen der Ejektionsfeder führen könnte.

Vorteilhafterweise ist hierzu an einem der beiden Elemente Basiselement und Schiebeelement ein Führungsschlitz oder eine Führungsnut ausgebildet, welche geradlinig in Injektionsrichtung verläuft, jedoch in dem proximalen Endbereich die Drehung des Schiebeelementes relativ zu dem Basiselement nachbildet, insbesondere bevorzugt sind Führungsschlitz oder Führungsnut somit in L-Form ausgebildet. An dem anderen der beiden Elemente ist bevorzugt ein Führungsfortsatz ausgebildet, insbesondere ein Zapfen, welcher in die vorgenannte Führungsnut bzw. den Führungsschlitz eingreift.

Das Schiebeelement weist bevorzugt Führungsschlitze für den Fortsatz des Kanülenoberteils auf, die derart angeordnet sind, dass nach Drehen des Schiebeelementes in den proximalen Endbereich das Kanülenoberteil entgegen der Injektionsrichtung verschiebbar ist. Hierdurch ist in konstruktiv einfacher Weise ein Ejektionsvorgang möglich, ohne dass das Schiebeelement entgegen der Injektionsrichtung bewegt werden muss.

In der vorteilhaften Ausgestaltung mit Vorsehen eines Ejektionselementes, insbesondere einer Ejektionsfeder erfolgt somit bei dem Ejektionsvorgang bevorzugt ein Verschieben des Kanülenoberteils mit Kanüle entgegen der Injektionsrichtung relativ zu dem Basiselement und relativ zu dem Schiebeelement, so dass während des Ejektionsvorgangs keine oder allenfalls eine geringfügige weitere Verschiebung zwischen Basiselement und Schiebeelement erfolgt.

Die vorgenannte Schräge ist bevorzugt an einem proximalen Ende der vorgenannten Führungsschlitze an dem Schiebeelement ausgebildet. Hierdurch ergibt sich ein konstruktiv einfacher Aufbau.

Vorteilhafterweise weist der Injektor eine Gegenkraftfeder auf, welche zwischen Basiselement und Schiebeelement einem Verschieben des Schiebelementes in Injektionsrichtung entgegenwirkend angeordnet ist. Hierdurch ist sichergestellt, dass der Injektionsvorgang nur mittels Druck auf das Schiebeelement gestartet wird und beispielsweise nicht bereits allein aufgrund der Gewichtskraft des Schiebeelementes erfolgt.

Weitere bevorzugte Merkmale und Ausführungsformen werden im Folgenden anhand eines Ausführungsbeispiels und den Figuren erläutert. Dabei zeigt:
- Figur 1: eine Seitenansicht eines Ausführungsbeispiels eines erfindungsgemäßen Injektors zum transkutanen Einführen eines Sensors in einen Patienten;
- Figur 2: Seitenansichten von Kanüle, Kanülenoberteil und Halteelement des Injektors;
- Figur 3: eine Draufsicht von oben der Elemente gemäß Figur 2;
- Figur 4: Schnittdarstellungen der Elemente aus Figur 2;
- Figur 5: eine Schnittdarstellung des Injektors vor dem Injektionsvorgang;
- Figur 6: eine Schnittdarstellung des Injektors nach Beenden des Injektionsvorgangs;
- Figur 7: eine weitere Schnittdarstellung am Ende des Injektionsvorgangs, wobei die Schnittebene senkrecht zu der Schnittebene gemäß Figur 6 steht;
- Figur 8: eine Detailansicht des Detektors mit einem Ejektionsfederhalteelement;
- Figur 9: eine Detailansicht des Injektors im proximalen Endbereich des Injektionsvorgangs;
- Figur 10: eine Seitenansicht des Injektors nach Beenden des Ejektionsvorgangs,
- Figur 11: eine Schnittdarstellung der Ansicht gemäß Figur 9 und
- Figur 12: eine Schnittdarstellung der Ansicht gemäß Figur 10.

Gleiche Bezugszeichen in den Figuren bezeichnen gleiche bzw. gleichwirkende Elemente.

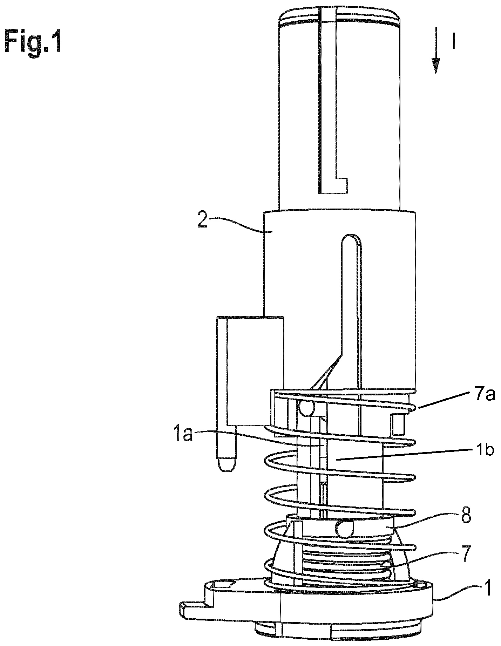

Figur 1 zeigt das Ausführungsbeispiel des erfindungsgemäßen Injektors in Seitenansicht. Der Injektor weist ein Basiselement 1 und ein Schiebeelement 2 auf. Das Schiebeelement ist in einer Injektionsrichtung I verschiebbar an dem Basiselement angeordnet.

Der Injektor kann zusätzlich ein Gehäuse aufweisen, welches an dem Basiselement angeordnet ist und das Basiselement sowie den unteren Teil, insbesondere die untere Hälfte des Schiebeelementes 2 gemäß Figur 1 umgibt. Aus Gründen der besseren Darstellbarkeit ist das Gehäuse in den Figuren nicht gezeigt.

Zur Verwendung des Injektors wird eine Basisplatte an die Haut des Patienten angeklebt und der Injektor mittels eines an der unteren Seite des Basiselementes ausgebildeten Bajonettverschlusses an die Basisplatte angebracht, so dass der Injektor lösbar an der Basisplatte und somit lösbar an dem Patienten angeordnet ist. Ebenso kann im Auslieferungszustand der Injektor bereits an der Basisplatte angebracht sein, so dass Injektor und Basisplatte an die Haut des Patienten angeklebt werden.

Das Gewebe des Patienten befindet sich somit bei allen Figuren an der unteren Seite, so dass in den Figuren die unteren Bereiche proximale Bereiche und die oberen Bereiche distale Bereiche zeigen.

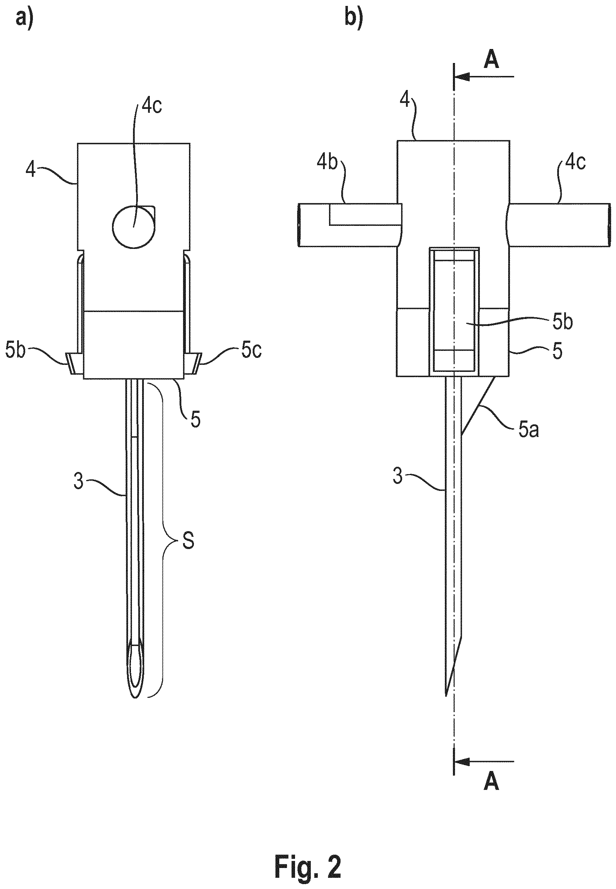

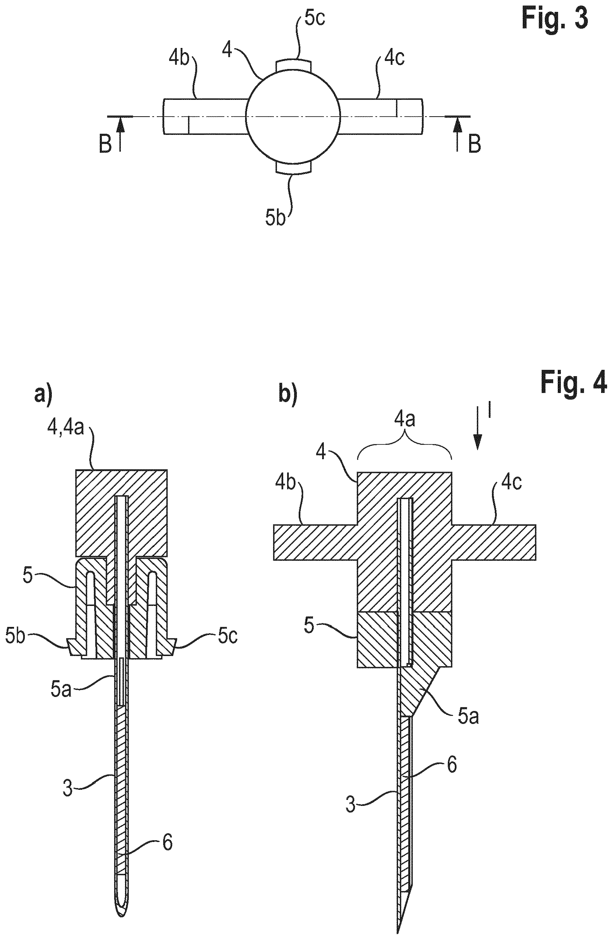

Das Basiselement weist einen in etwa als Hohlzylinder ausgebildeten Bereich auf, welcher eine Kanüle 3 mit einem Kanülenoberteil 4 und ein Halteelement 5 annähernd umschließt. Diese Elemente sind in den Figuren 2 bis 4 separat dargestellt:
Die Kanüle 3 ist an ihrem distalen Ende in einem Zentralelement 4a (siehe Figur 4b) des Kanülenoberteils 4 eingebettet und fest mit diesem verbunden. Unterhalb des Kanülenoberteils 4 ist das Halteelement 5 angeordnet, welches im distalen Bereich eine Einbuchtung aufweist, in welche das Kanülenoberteil 4 eingreift, wobei das Halteelement 5 mit leichtem Presssitz an dem Kanülenoberteil 4 angeordnet ist.

Das Kanülenoberteil 4 weist weiterhin zwei an gegenüberliegenden Seiten angeordnete Fortsätze 4b, 4c auf, welche sich senkrecht zur Längserstreckung der Kanüle 3 und somit senkrecht zur Injektionsrichtung I erstrecken.

Figur 2a zeigt hierbei eine Seitenansicht mit Draufsicht auf die Stirnseite des Fortsatzes 4c und Figur 2b zeigt eine Seitenansicht mit in der Zeichenebene liegenden Längserstreckung der Fortsätze 4b und 4c.

Figur 3 zeigt eine Draufsicht von oben auf das Kanülenoberteil 4. Figur 4a zeigt einen Schnitt gemäß der Schnittlinie A-A in Figur 2b, wobei die Schnittebene senkrecht zur Zeichenebene der Figur 2a steht. Figur 4b zeigt einen Schnitt gemäß der Schnittlinie B-B in Figur 3, wobei ebenfalls die Schnittebene senkrecht zur Zeichenebene gemäß Figur 3 steht.

Wie beispielsweise in Figur 2a ersichtlich, weist die Kanüle 3 in einem proximalem Bereich S einen Schlitz auf. Figur 2a zeigt die Draufsicht von vorne auf den Schlitz der Kanüle 3. In der Kanüle 3 ist ein Sensor 6 angeordnet, wie beispielsweise in den Figuren 4a und 4b ersichtlich.

Dieser Sensor soll transkutan in das Gewebe des Patienten mittels des Injektors eingebracht werden, um mittels eines als Detektor ausgebildeten Detektionselementes/Detektionseinheit optisch Messwerte zu ermitteln. Die Grundprinzipien einer solchen optische Messung sind in WO2016128334A1 und WO2006092317A1 beschrieben.

Ebenso ist das Einbringen anderer Sensoren, insbesondere von Sensoren mit Elektroden zur elektrischen Erfassung von Messwerten in gleicher Weise möglich.

Wie insbesondere in den Figuren 2b, 4a und 4b ersichtlich, weist das Halteelement 5 eine Nocke 5a auf, welche im Bereich des distalen Endes des Sensors 6 durch den Schlitz der Kanüle 3 in diese eingreift und somit am oberen, distalen Ende des Sensors 6 anliegt (siehe Figur 4b).

Das Halteelement 5 weist weiterhin als Rastnasen ausgebildete Festlegemittel 5b und 5c auf, so dass am Ende eines Injektionsvorgangs das Halteelement 5 selbsttätig an dem Basiselement arretierbar ist:

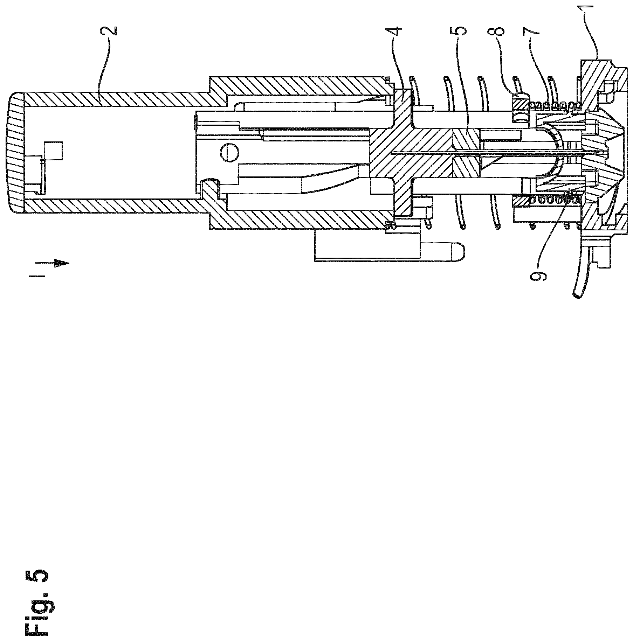

Figur 5 zeigt eine Schnittdarstellung des Injektors, wobei die Schnittebene durch die Zentralachse der Kanüle 3 und somit durch die Injektionsachse verläuft. Dargestellt ist der Zustand vor dem Injektionsvorgang, welcher den Auslieferungszustand darstellt: Kanüle und Sensor befinden sich innerhalb des Injektors, insbesondere innerhalb des Basiselementes 1.

Kanüle 3 mit Sensor 6, Halteelement 5 und Zentralelement 4a des Kanülenoberteils 4 sind innerhalb eines in etwa zylindrisch ausgebildeten Bereichs des Basiselementes 1 angeordnet. Dieser zylindrische Bereich weist geradlinig in Injektionsrichtung I verlaufende Führungsschlitze auf, von denen ein Führungsschlitz 1a in Figur 1 ersichtlich ist.

Die Fortsätze 4b und 4c des Kanülenoberteils 4 durchdringen den in etwa zylindrischen Bereich des Basiselements 1. Wie in Figur 5 ersichtlich, greift das Schiebeelement 2 außerhalb des zylindrischen Bereichs des Basiselementes 1 an den Fortsätzen 4b und 4c an. Das Schiebeelement 2 wird somit an einer Außenwand des zylindrischen Bereichs des Basiselements 1 in Injektionsrichtung verschiebbar geführt und Zentralelement 4a des Kanülenoberteils 4 sowie Halteelement 5 werden an der Innenseite des zylindrischen Bereichs des Basiselementes 1 in Injektionsrichtung verschiebbar geführt. Der zylindrische Bereich des Basiselements 1 bildet somit eine Führungswand 1b für diese Elemente.

Drückt der Benutzer nun das Schiebeelement 2 in Injektionsrichtung nach unten, so wird die Kraft über die Fortsätze 4b und 4c auf das Zentralelement 4a und somit auf die Kanüle 3 übertragen. Ebenso wird die Kraft über das Zentralelement 4a auf das Halteelement 5 übertragen, so dass diese Elemente sowie der Sensor 6 in Injektionsrichtung bewegt werden.

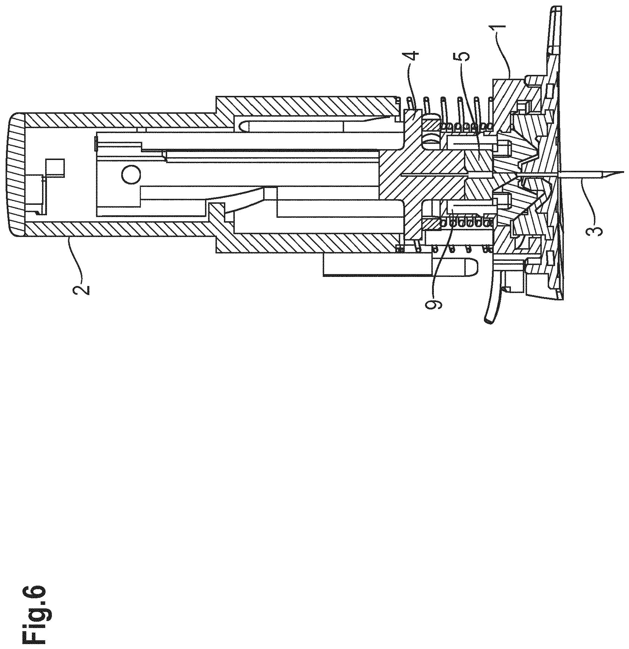

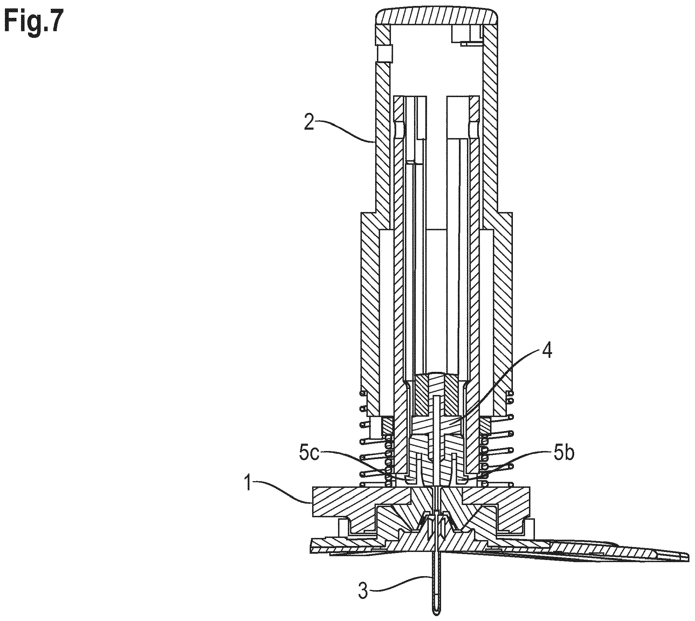

Figur 6 zeigt das Ende des Injektionsvorgangs: Das Schiebeelement 2 ist vollständig heruntergedrückt, die Kanüle 3 mit dem Sensor 6 ist transkutan in das Gewebe des Patienten eingedrungen und die als Rastnasen ausgebildeten Festlegemittel 5b und 5c sind in entsprechende Ausnehmungen des Basiselements 1 eingerastet, wie in Figur 7 ersichtlich.

Am Ende des Injektionsvorgangs ist somit das Halteelement 5 selbsttätig an dem Basiselement 1 festgelegt.

Bei dem nachfolgenden Ejektionsvorgang wird das Kanülenoberteil 4 entgegen der Injektionsrichtung I nach oben bewegt, so dass die Kanüle 3 aus dem Gewebe des Patienten herausgezogen wird. Da das Halteelement 5 jedoch an dem Basiselement 1 festgelegt ist, vollzieht das Halteelement diese Bewegung entgegen der Injektionsrichtung nicht mit. Ein eventuell vorhandener Presssitz zwischen Halteelement 5 und Kanülenoberteil 4 wird durch die Festlegung mittels der Rastnasen überwunden. Da weiterhin die Nocke 5a des Halteelements 5 in den Schlitz der Kanüle 3 an dem distalen Ende des Sensors in die Kanüle eingreift, wird hierdurch verhindert, dass bei einem Herausziehen der Kanüle 3 auch der Sensor aus dem Gewebe des Patienten herausgezogen wird. Insbesondere kann auch ein Anhaften oder eine Reibung zwischen Sensor und Kanüle hierdurch überwunden werden.

Nach Abschluss des Ejektionsvorgangs wird der Injektor von der zuvor genannten Basisplatte entfernt, so dass lediglich Basisplatte und Sensor 6 an dem Patienten verbleiben. Halteelement 5 und Sensor 5 sind somit als separate Einheiten ausgebildet.

Der Injektor gemäß dem vorliegenden Ausführungsbeispiel weist weiterhin ein als Ejektionsfeder 7 ausgebildetes Ejektionselement und ein als Ejektionsfederhalteelement 8 ausgebildetes Arretierungselement für das Ejektionselement auf. Wie in Figur 1 ersichtlich, ist im Auslieferungszustand die Ejektionsfeder 7 komprimiert zwischen Basiselement 1 und Ejektionsfederhalteelement 8 angeordnet. Die Ejektionsfeder 7 umschließt den zylindrischen Bereich des Basiselementes 1 in einem proximalen Bereich.

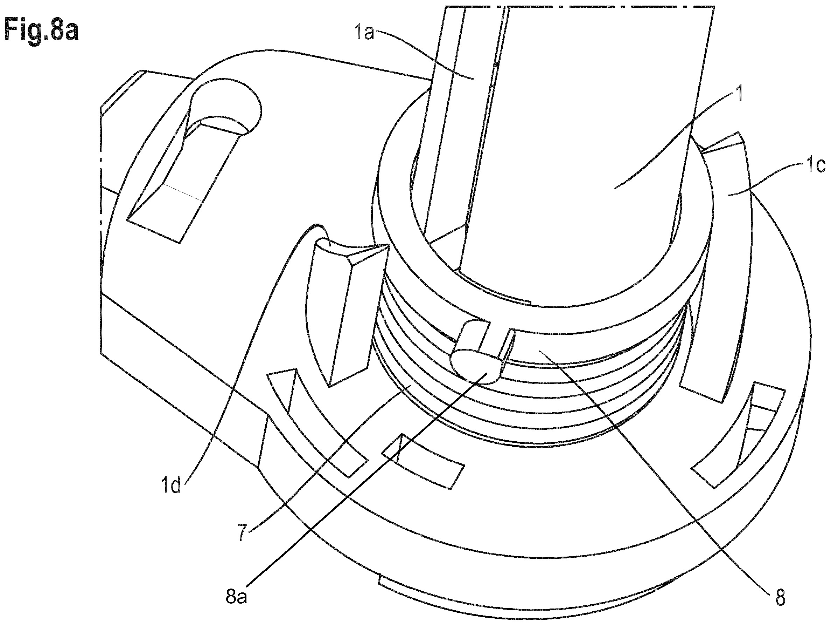

Das Ejektionsfederhalteelement 8 ist im Wesentlichen ringförmig ausgebildet und weist sowohl an der Innenseite, als auch an der Außenseite einen Zapfen auf. Mittels des inneren Zapfens ist das Ejektionsfederhalteelement an einer senkrecht zur Injektionsrichtung verlaufenden Führung des Basiselementes lösbar festgelegt, siehe Figur 8a. In dem durch einen Kreis in Figur 8a markierten Bereich greift der innere Zapfen des Ejektionsfederhalteelements 8 in eine Führung an der Außenwand des Basiselementes 1 ein, so dass in diesem Zustand keine Expansion der Ejektionsfeder 7 möglich ist. Wie nachfolgend näher erläutert, sind Schiebeelement 2 und Ejektionsfederhalteelement 8 derart zusammenwirkend ausgebildet, dass am Ende des Injektionsvorgangs eine Drehung des Ejektionsfederhalteelementes 8 erfolgt, so dass der Zapfen des Ejektionsfederhalteelementes 8 in der Darstellung gemäß Figur 8a nach links gedreht wird und so in den Bereich des Führungsschlitzes 1a des Basiselementes 1 gelangt, so dass eine Expansion der Ejektionsfeder 7 möglich ist.

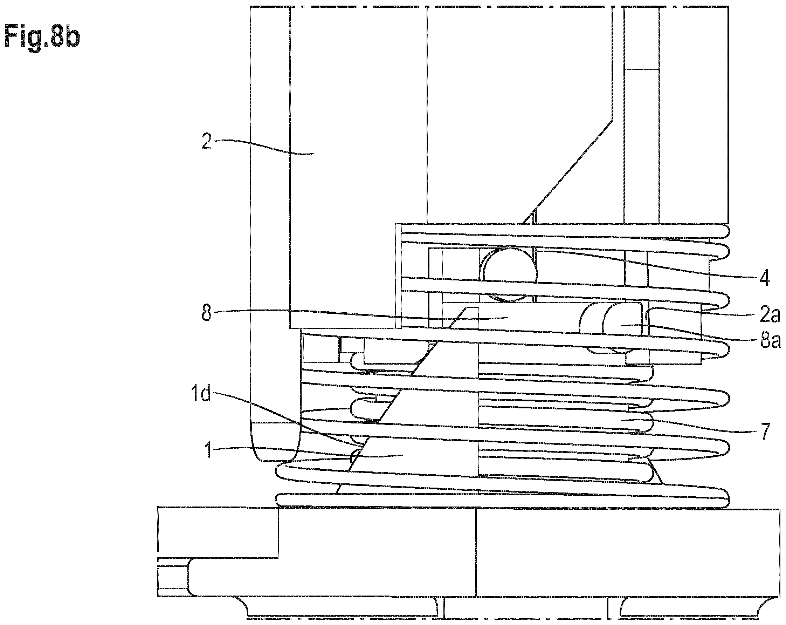

Wie in den Figuren 8a und 8b ersichtlich, weist das Basiselement in einem proximalen Bereich zwei als Schräge, d. h. schräge Flächen, ausgebildete Führungen 1c und 1d welche am Ende des Injektionsvorgangs mit korrespondierenden Anlageflächen des Schiebeelementes 2 in Kontakt gelangen. Wird in diesem Endbereich das Schiebeelement weiter heruntergedrückt, so erfolgt aufgrund der Schrägen 1c und 1d eine Drehung des Schiebeelementes relativ zu dem Basiselement um die Injektionsachse. Zur besseren Verdeutlichung sind in Figur 8a Elemente wie beispielsweise das Schiebeelement nicht dargestellt.

Wie in Figur 8b ersichtlich, weist das Ejektionsfederhalteelement 8 einen als Zapfen ausgebildeten äußeren Fortsatz 8a auf, welcher bei Drehen des Schiebeelementes 2 mit einer korrespondierenden Führungsfläche 2a des Schiebeelementes 2 in Kontakt kommt, so dass eine Drehung des Ejektionsfederhalteelementes 8 wie zuvor beschrieben erfolgt.

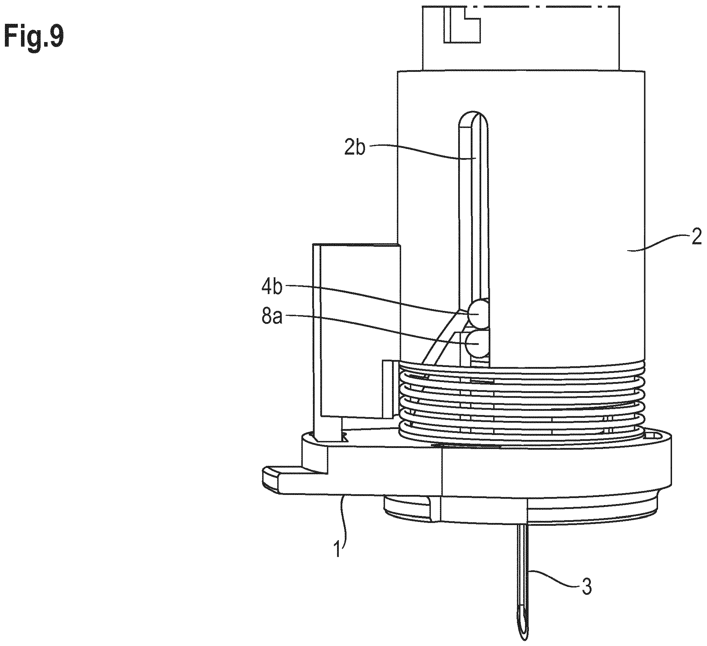

Figur 9 zeigt den Zustand mit vollständig nach unten gedrücktem Schiebeelement 2, so dass auch die Drehung des Schiebeelementes 2 relativ zu dem Basiselement 1 beendet ist. Wie ersichtlich, befinden sich in diesem Endzustand des Injektionsvorgangs sowohl der äußere Zapfen 8a des Ejektionsfederhalteelementes 8, als auch der Fortsatz 4b des Kanülenoberteils 4 im Bereich eines Führungsschlitzes 2b des Schiebeelementes 2. Auf der gegenüberliegenden Seite (nicht ersichtlich) befindet sich entsprechend der Fortsatz 4c des Kanülenoberteils 4 in einem radial gegenüberliegend angeordneten Führungsschlitz des Schiebeelementes 2.

In diesem Zustand besteht somit für die Fortsätze 4b und 4c sowie für den äußeren Zapfen 8a keine Begrenzung hinsichtlich einer Bewegung entgegen der Injektionsrichtung. Im Ergebnis erfolgt eine Expansion der Ejektionsfeder 7, wodurch Ejektionsfederhalteelement 8 und Kanülenoberteil 4 in einem Ejektionsvorgang entgegen der Injektionsrichtung nach oben gedrückt werden. Das Halteelement 5 hingegen ändert aufgrund der eingerasteten Halteelemente die Position nicht.

Es erfolgt somit ein Herausziehen der Kanüle 3 aus dem Gewebe des Patienten, wobei der Sensor 6 durch die Nocke 5a des Haltelementes 5 an einem Herausziehen gehindert wird.

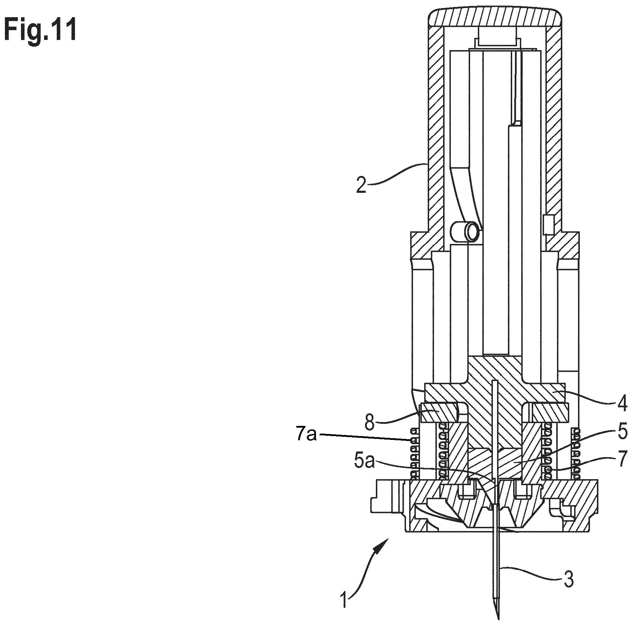

In Figur 11 ist die Konstellation aus Figur 9 als Schnittbild dargestellt, wobei die Injektionsachse innerhalb der Schnittebene liegt und der Schnitt entlang der Schnittlinie B gemäß Figur 3 verläuft. Hier ist insbesondere die komprimierte Ejektionsfeder 7 ersichtlich, welche an dem Halteelement 8 einerseits und dem Basiselement 1 andererseits angreift. Konzentrisch zu der Ejektionsfeder 7, jedoch mit größerem Radius ist eine Gegenkraftfeder 7a angeordnet, welche an dem Basiselement 1 einerseits und Schiebeelement 2 andererseits angreift. Auch diese Gegenkraftfeder ist in dieser Konstellation vollständig komprimiert. Die Gegenkraftfeder dient insbesondere dem Zweck, ein Herabsinken des Schiebeelementes 2 aufgrund der Schwerkraft zu vermeiden sowie dem Benutzer bei dem Injektionsvorgang eine in etwa konstante Gegenkraft zu bieten, um eine gleichmäßige Injektion zu ermöglichen, insbesondere eine gleichmäßige Geschwindigkeit des Eindringens der Kanüle 3 in den Patienten.

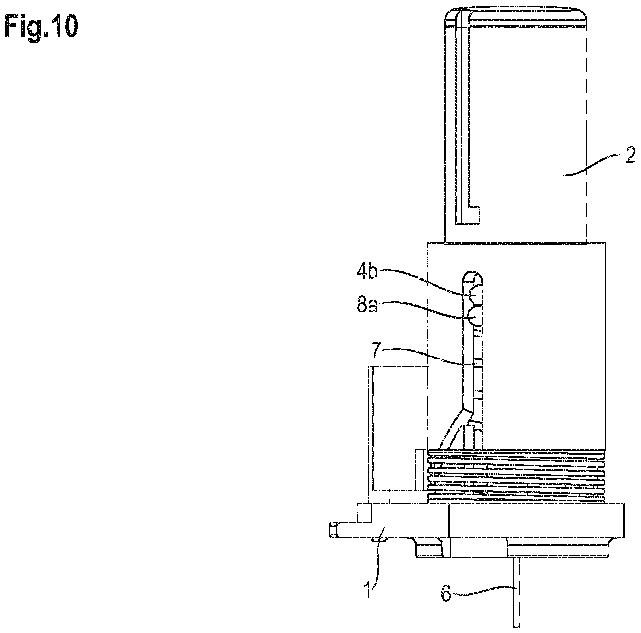

In Figur 10 ist die Ansicht gemäß Figur 9 dargestellt, jedoch nach Beenden des Ejektionsvorgangs:
Wie zuvor beschrieben, wird am Ende des Injektionsvorgangs das Halteelement 8 um die Injektionsachse gedreht, so dass es aus einer Festlegestellung, bei welcher keine Bewegung des Halteelementes in Ejektionsrichtung, d. h. entgegen der Injektionsrichtung I möglich ist in eine Ejektionsstellung überführt wird, in welcher eine Bewegung in Ejektionsrichtung erfolgen kann.

Wie bereits zu Figur 8a beschrieben, weist das Halteelement 8 an der Innenseite einen Zapfen auf, welcher gemäß der in Figur 8a dargestellten Festlegestellung in einem waagerecht verlaufenden Schlitz des Basiselementes 1 angeordnet ist, so dass keine Bewegung in Ejektionsrichtung (in Figur 8a nach oben) möglich ist.

Am Ende des Injektionsvorgangs erfolgt eine Drehung des Halteelementes 8 um die Injektionsachse, gemäß Figur 8a im Uhrzeigersinn, so dass der innere Zapfen des Halteelementes 8 in der Flucht des Führungsschlitzes 1a des Basiselementes 1 zu liegen kommt. Hierdurch ist somit eine Bewegung des Führungselementes 8 in Ejektionsrichtung (nach oben) möglich. Diese Drehung des Halteelementes 8 erfolgt, da am Ende des Injektionsvorgangs aufgrund der Schrägen 1d und 1c des Basiselementes 1 und der korrespondierenden Schrägen des Schiebeelementes 2 eine Drehung des Schiebeelementes um die Injektionsachse (vorliegend im Uhrzeigersinn) erfolgt und durch die Fläche 2a des Schiebeelementes 2, welche an dem äußeren Zapfen des Halteelementes 8 angreift, die Drehung auf das Halteelement 8 übertragen wird.

Befindet sich das Halteelement 8 in Ejektionsstellung, so ist eine Expansion der Expansionsfeder 7 möglich: Hierdurch wird das Halteelement 8 nach oben gedrückt und diese Bewegung auf das Kanülenoberteil 4 und somit auch die Kanüle 3 übertragen, so dass die Kanüle 3 in einem Ejektionsvorgang aus dem Patienten herausgezogen wird. Aufgrund des zuvor beschriebenen Halteelementes 5 verbleibt der Sensor 6 jedoch transkutan in dem Patienten.

Figur 10 zeigt nun die Situation nach Beenden des Ejektionsvorgangs: Die Ejektionsfeder 7 befindet sich in einem expandierten Zustand, Halteelement 8 und Kanülenoberteil 4 (sowie Kanüle 3) sind nach oben geschoben und befinden sich innerhalb des Basiselementes 1. Die Position des Schiebeelementes 2 ist jedoch unverändert, so dass nach wie vor die außenliegende Gegenkraftfeder sich in einem komprimierten Zustand befindet.

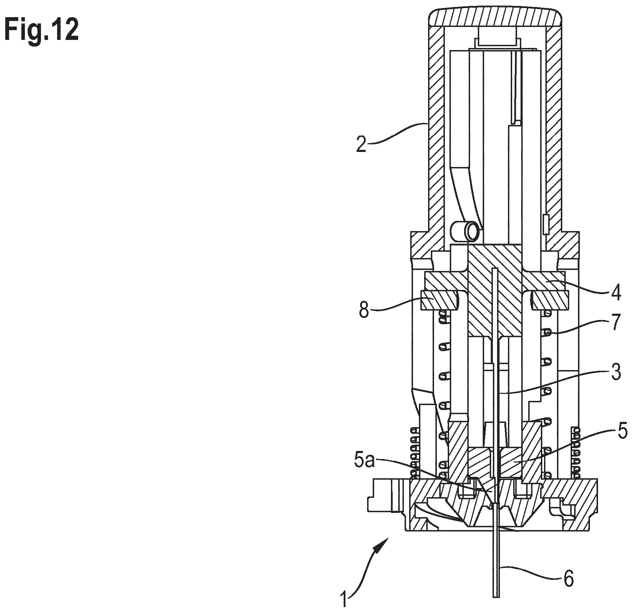

In Figur 12 ist ein Schnitt analog zu Figur 11 dargestellt, jedoch am Ende des Ejektionsvorgangs gemäß Figur 10.

Der Injektor gemäß dem vorliegenden Ausführungsbeispiel weist weiterhin eine Kanülenführung 9 für die Kanüle 3 auf, wie insbesondere in den Figuren 5 und 6 ersichtlich:
Die Kanülenführung 9 dient dazu, die Kanüle insbesondere während des Injektionsvorgangs, jedoch auch während des Ejektionsvorgangs zu führen, um ein Verkippen oder seitliches Verrutschen zu vermeiden. Hierzu ist die Kanülenführung 9 an zwei gegenüberliegenden Seiten an dem Basiselement 1 angeordnet und weist mittig eine elastische Führungsfläche mit einer Öffnung auf, welche durch die Kanüle 3 durchdrungen wird und die Kanülenführung 9 in zwei Hälften unterteilt. In Figur 5 ist der Zustand vor Beginn der Injektion dargestellt. Hier wird die Kanüle 3 in einem proximalen Bereich durch die Kanülenführung 9 geführt, so dass bei der nachfolgenden Injektion ein Verkippen oder seitliches Verschieben vermieden wird.

Nach Beenden des Injektionsvorgangs erreicht das Halteelement 5 und das Kanülenoberteil 4 den Bereich der Kanülenführung 9. Aufgrund der Zweiteilung der Kanülenführung 9 können die elastischen Elemente der Kanülenführung 9 durch das Halteelement 5 nach rechts und links gedrückt werden. Dies ist in Figur 6 ersichtlich.

## Patentansprüche

1. Injektor zum transkutanen Einführen eines Sensors in einen Patienten, mit einer Kanüle (3), einem in der Kanüle (3) angeordneten Sensor (6), einem Basiselement (1), einem in einer Injektionsrichtung verschiebbar an dem Basiselement (1) angeordneten Schiebeelement (2) zum transkutanen Einführen der Kanüle (3) mit dem Sensor (6) in den Patienten in einem Injektionsvorgang,
wobei die Kanüle (3) zumindest in einem proximalen Bereich (S) einen Schlitz in einer Längsrichtung der Kanüle (3) aufweist,
wobei
der Injektor ein verschiebbar an dem Basiselement (1) angeordnetes Halteelement (5) aufweist, um in einem Ejektionsvorgang bei einem Herausziehen der Kanüle (3) aus dem Patienten ein Herausziehen des Sensors mittels des Halteelements zu verhindern,
wobei das Halteelement (5) im Bereich eines distalen Endes des Sensors durch den Schlitz in die Kanüle (3) eingreift,
wobei Halteelement (5) und/oder Basiselement (1) zumindest ein Festlegemittel (5b, 5c) aufweisen, so dass im Zustand der in den Patienten transkutan eingeführten Kanüle (3) das Halteelement (5) mittelbar oder bevorzugt unmittelbar an dem Basiselement (1) selbsttätig arretierbar ist,
**dadurch gekennzeichnet,**
**dass** Halteelement (5) und Sensor (6) trennbar, bevorzugt als separate Einheiten ausgebildet sind, so dass bei Entfernen des Injektors auch das Halteelement entfernt wird, ohne den Sensor herauszuziehen.

2. Injektor nach Anspruch 1, **dadurch gekennzeichnet,**
**dass** das Festlegemittel (5b, 5c) als Rastelement ausgebildet ist und eines der beiden Elemente Halteelement (5) und Basiselement (1) das Rastelement aufweist und das andere der beiden Elemente korrespondierende Vertiefungen für das Rastelement aufweist.

3. Injektor nach einem der vorangegangenen Ansprüche,
**dadurch gekennzeichnet,**
**dass** die Kanüle (3) mit ihrem distalen Ende an einem verschiebbar an dem Basiselement (1) angeordneten Kanülenoberteil (4) angeordnet ist und dass Schiebelement und Kanülenoberteil (4) derart zusammenwirkend ausgebildet sind, dass bei dem Inkjektionsvorgang das Kanülenoberteil (4) mittels des Schiebeelements (2) in eine Injektionsrichtung verschiebbar ist.

4. Injektor nach Anspruch 3,
**dadurch gekennzeichnet,**
**dass** das Halteelement (5) derart mit dem Kanülenoberteil (4) zusammenwirkend ausgebildet ist, dass bei dem Injektionsvorgang das Halteelement (4) mittels des Kanülenoberteils (4) in Injektionsrichtung verschiebbar ist.

5. Injektor nach einem der vorangegangenen Ansprüche 3 bis 4,
**dadurch gekennzeichnet,**
**dass** das Kanülenoberteil (4) ein Zentralelement (4a), an welchem die Kanüle (3) angeordnet und mindestens einen Führungsfortsatz (4b, 4c) aufweist und das Basiselement (1) mindestens eine Führungswand (1b) mit einem Führungsschlitz (1a) für den Führungsfortsatz (4b, 4c) des Kanülenoberteil (4) aufweist, zur Führung des Kanülenoberteils in Injektionsrichtung, insbesondere, dass der Führungsfortsatz (4b, 4c) die Führungswand (1b) des Basiselements (1) durchdringt und das Schiebelement an der dem Zentralelement (4a) des Kanülenoberteils abgewandten Seite der Führungswand (1b) an dem Führungsfortsatz (4b, 4c) angreifend ausgebildet ist.

6. Injektor nach einem der vorangegangenen Ansprüche,
**dadurch gekennzeichnet,**
**dass** der Injektor eine Ejektionsfeder (7) und ein Ejektionsfederhalteelement (8) aufweist, wobei das Ejektionsfederhalteelement (8) an dem Basiselement (1) festlegbar ausgebildet ist, so dass die Ejektionsfeder (7) mittels des Ejektionsfederhalteelements in einem komprimierten oder gedehnten Zustand festlegbar ist.

7. Injektor nach Anspruch 6,
**dadurch gekennzeichnet,**
**dass** das Schiebeelement (2) mit dem Ejektionsfederhalteelement zusammenwirkend ausgebildet ist, so dass mittels des Schiebeelements das Ejektionsfederhalteelement (8) am Ende des Injektionsvorgangs aus einer Festlegestellung lösbar ist, insbesondere durch Drehung des Ejektionsfederhalteelements um eine Injektionsachse.

8. Injektor nach einem der Ansprüche 6 bis 7,
**dadurch gekennzeichnet,**
**dass** Schiebelement (2) und Ejektionsfederhalteelement (8) um eine gemeinsame Injektionsachse drehbar an dem Basiselement (1) angeordnet sind.

9. Injektor nach einem der vorangegangenen Ansprüche,
**dadurch gekennzeichnet,**
**dass** Basiselement (1) und/oder Schiebelement zumindest eine Schräge (1c, 1d) aufweisen, die derart angeordnet ist, dass beim Injektionsvorgang in einem proximalen Endbereich bei Verschieben des Schiebelementes in Injektionsrichtung eine Drehung des Schiebelements relativ zu dem Basiselement (1) erfolgt.

10. Injektor nach den Ansprüchen 7 und 9,
**dadurch gekennzeichnet,**
**dass** Schiebeelement (2) und Ejektionsfederhalteelement (8) korrespondierende Anlageflächen aufweisen, die derart angeordnet sind, dass durch Drehung des Schiebelements das Ejektionselement aus einer Festlegestellung lösbar ist, insbesondere, dass das Ejektionsfederhalteelement (8) einen Fortsatz und das Schiebeelement (2) eine korrespondierende Führungsfläche (2a) aufweist.

11. Injektor nach einem der Ansprüche 9 bis 10,
**dadurch gekennzeichnet,**
**dass** Basiselement (1) und Schiebelement korrespondierende Führungselemente aufweisen, die derart ausgebildet und angeordnet sind, dass nur in dem proximalen Endbereich das Schiebeelement (2) relativ zu dem Basiselement (1) drehbar ist.

12. Injektor nach Anspruch 4 und 9,
**dadurch gekennzeichnet,**
**dass** das Schiebelement Führungsschlitze für den Fortsatz des Kanülenoberteils aufweist die derart angeordnet sind, dass nach Drehen des Schiebelementes in dem proximalen Endbereich das Kanülenoberteil entgegen der Injektionsrichtung verschiebbar ist.

13. Injektor nach Anspruch 12,
**dadurch gekennzeichnet,**
**dass** die Schräge (1c, 1d) an einem proximalen Ende der Führungsschlitze an dem Schiebelement ausgebildet ist.

14. Injektor nach einem der vorangegangenen Ansprüche,
**dadurch gekennzeichnet,**
**dass** der Injektor eine Gegenkraftfeder (7a) aufweist, welche zwischen Basiselement (1) und Schiebelement einem Verschieben des Schiebelementes in Injektionsrichtung entgegenwirkend angeordnet ist.

## Claims

1. Injector for transcutaneous insertion of a sensor into a patient, having a cannula (3), a sensor (6) arranged in the cannula (3), a base element (1), a sliding element (2) arranged on the base element (1) so as to be displaceable in an injection direction for transcutaneous insertion of the cannula (3) with the sensor (6) into the patient in an injection operation,
wherein the cannula (3) has, at least in a proximal region (S), a slot in a longitudinal direction of the cannula (3),
wherein
the injector has a holding element (5) displaceably arranged on the base element (1) in order that, in an ejection operation, the holding element prevents the sensor from being withdrawn when the cannula (3) is withdrawn from the patient,
wherein the holding element (5) extends through the slot into the cannula (3) in the region of a distal end of the sensor,
wherein holding element (5) and/or base element (1) have at least one fixing means (5b, 5c) so that, in the state in which the cannula (3) has been inserted transcutaneously into the patient, the holding element (5) is automatically indirectly or, preferably, directly lockable on the base element (1),
**characterised in that**
holding element (5) and sensor (6) are configured so as to be separable, preferably being formed as separate units, so that on removal of the injector the holding element is also removed without withdrawal of the sensor.

2. Injector according to claim 1,
**characterised in that**
the fixing means (5b, 5c) is in the form of a detent element and one of the two elements holding element (5) and base element (1) has the detent element and the other of the two elements has complementary recesses for the detent element.

3. Injector according to either one of the preceding claims,
**characterised in that**
the cannula (3) is arranged with its distal end on a cannula upper part (4) which is arranged so as to be displaceable on the base element (1); and the sliding element and cannula upper part (4) are designed to co-operate in such a way that, during the injection operation, the cannula upper part (4) is displaceable in an injection direction by means of the sliding element (2).

4. Injector according to claim 3,
**characterised in that**
the holding element (5) is designed to co-operate with the cannula upper part (4) in such a way that, during the injection operation, the holding element (4) is displaceable in the injection direction by means of the cannula upper part (4).

5. Injector according to either one of preceding claims 3 and 4,
**characterised in that**
the cannula upper part (4) has a central element (4a), on which the cannula (3) is arranged, and at least one guide projection (4b, 4c), and the base element (1) has at least one guide wall (1b) having a guide slot (1a) for the guide projection (4b, 4c) of the cannula upper part (4), for guiding the cannula upper part in the injection direction; especially, the guide projection (4b, 4c) passes through the guide wall (1b) of the base element (1) and the sliding element is designed to act on the guide projection (4b, 4c) on the side of the guide wall (1b) that faces away from the central element (4a) of the cannula upper part.

6. Injector according to any one of the preceding claims,
**characterised in that**
the injector has an ejection spring (7) and an ejection spring holding element (8), wherein the ejection spring holding element (8) is designed to be fixable on the base element (1), so that the ejection spring (7) is fixable in a compressed or expanded state by means of the ejection spring holding element.

7. Injector according to claim 6,
**characterised in that**
the sliding element (2) is designed to co-operate with the ejection spring holding element so that, by means of the sliding element, the ejection spring holding element (8) is releasable from a fixing position at the end of the injection operation, especially by rotation of the ejection spring holding element about an injection axis.

8. Injector according to either one of claims 6 and 7,
**characterised in that**
sliding element (2) and ejection spring holding element (8) are arranged on the base element (1) so as to be rotatable about a common injection axis.

9. Injector according to any one of the preceding claims,
**characterised in that**
base element (1) and/or sliding element have at least one sloping surface (1c, 1d) which is arranged in such a way that, during the injection operation, the sliding element rotates relative to the base element (1) in a proximal end region on displacement of the sliding element in the injection direction.

10. Injector according to claims 7 and 9,
**characterised in that**
sliding element (2) and ejection spring holding element (8) have complementary contact faces which are arranged in such a way that, by rotation of the sliding element, the ejection element is releasable from a fixing position; especially, the ejection spring holding element (8) has a projection and the sliding element (2) has a complementary guide face (2a).

11. Injector according to either one of claims 9 and 10,
**characterised in that**
base element (1) and sliding element have complementary guide elements which are configured and arranged in such a way that the sliding element (2) is rotatable relative to the base element (1) only in the proximal end region.

12. Injector according to claim 4 and 9,
**characterised in that**
the sliding element has guide slots for the projection of the cannula upper part, which guide slots are arranged in such a way that, after rotation of the sliding element in the proximal end region, the cannula upper part is displaceable in a direction opposite to the injection direction.

13. Injector according to claim 12,
**characterised in that**
the sloping surface (1c, 1d) is formed on the sliding element at a proximal end of the guide slots.

14. Injector according to any one of the preceding claims,
**characterised in that**
the injector has a counter-force spring (7a) which is arranged between base element (1) and sliding element so as to counteract displacement of the sliding element in the injection direction.

## Revendications

1. Injecteur pour l'introduction transcutanée d'un capteur dans un patient, comprenant une canule (3), un capteur (6) disposé dans la canule (3), un élément de base (1), un élément coulissant (2) disposé sur l'élément de base (1) de manière à pouvoir coulisser dans une direction d'injection pour l'introduction transcutanée de la canule (3) avec le capteur (6) dans le patient lors d'une opération d'injection,
dans lequel la canule (3) présente au moins dans une zone proximale (S) une fente dans une direction longitudinale de la canule (3),
dans lequel
l'injecteur présente un élément de maintien (5) disposé de manière à pouvoir coulisser sur l'élément de base (1) pour empêcher au moyen de l'élément de maintien un retrait du capteur lors d'une opération d'éjection lorsque la canule (3) est retirée du patient,
dans lequel l'élément de maintien (5) vient en prise avec la canule (3) par la fente dans la zone d'une extrémité distale du capteur,
dans lequel l'élément de maintien (5) et/ou l'élément de base (1) présentent au moins un moyen de fixation (5b, 5c) de telle sorte qu'à l'état de la canule (3) introduite de manière transcutanée dans le patient, l'élément de maintien (5) puisse être arrêté de manière autonome indirectement ou de manière préférée directement sur l'élément de base (1),
**caractérisé en ce que**
l'élément de maintien (5) et le capteur (6) sont réalisés de manière à pouvoir être séparés, de manière préférée en tant qu'unités séparées, de telle sorte que lorsque l'injecteur est enlevé, l'élément de maintien puisse enlevé également sans retirer le capteur.

2. Injecteur selon la revendication 1,
**caractérisé en ce que**
le moyen de fixation (5b, 5c) est réalisé en tant qu'élément d'enclenchement et un des deux éléments, l'élément de maintien (5) et l'élément de base (1), présente l'élément d'enclenchement et l'autre des deux éléments présente des renfoncements correspondants pour l'élément d'enclenchement.

3. Injecteur selon l'une des revendications précédentes,
**caractérisé en ce que**
la canule (3) est disposée avec son extrémité distale en une partie supérieure de canule (4) disposée de manière à pouvoir coulisser sur l'élément de base (1), et l'élément coulissant et la partie supérieure de canule (4) sont réalisés de manière à coopérer de telle manière que lors de l'opération d'injection, la partie supérieure de canule (4) puisse être coulissée dans une direction d'injection au moyen de l'élément coulissant (2).

4. Injecteur selon la revendication 3,
**caractérisé en ce que**
l'élément de maintien (5) est réalisé de manière à coopérer avec la partie supérieure de canule (4) de telle manière que lors de l'opération d'injection, l'élément de maintien (4) puisse être coulissé dans la direction d'injection au moyen de la partie supérieure de canule (4).

5. Injecteur selon l'une des revendications précédentes 3 à 4,
**caractérisé en ce que**
la partie supérieure de canule (4) présente un élément central (4a) sur lequel la canule (3) est disposée et au moins un prolongement de guidage (4b, 4c), et l'élément de base (1) présente au moins une paroi de guidage (1b) avec une fente de guidage (la) pour le prolongement de guidage (4b, 4c) de la partie supérieure de canule (4) pour guider la partie supérieure de canule dans la direction d'injection, en particulier le prolongement de guidage (4b, 4c) traverse la paroi de guidage (1b) de l'élément de base (1) et l'élément coulissant est réalisé de manière à s'engager sur le prolongement de guidage (4b, 4c) du côté de la paroi de guidage (1b) opposé à l'élément central (4a) de la partie supérieure de canule.

6. Injecteur selon l'une des revendications précédentes,
**caractérisé en ce que**
l'injecteur présente un ressort d'éjection (7) et un élément de maintien de ressort d'éjection (8), dans lequel l'élément de maintien de ressort d'éjection (8) est réalisé de manière à pouvoir être fixé sur l'élément de base (1) de telle sorte que le ressort d'éjection (7) puisse être fixé au moyen de l'élément de maintien de ressort d'éjection dans un état comprimé ou dilaté.

7. Injecteur selon la revendication 6,
**caractérisé en ce que**
l'élément coulissant (2) est réalisé de manière à coopérer avec l'élément de maintien de ressort d'éjection de telle sorte que l'élément de maintien de ressort d'éjection (8) puisse être libéré d'une position de fixation à la fin de l'opération d'injection au moyen de l'élément coulissant, en particulier par rotation de l'élément de maintien de ressort d'éjection autour d'un axe d'injection.

8. Injecteur selon l'une des revendications 6 à 7,
**caractérisé en ce que**
l'élément coulissant (2) et l'élément de maintien de ressort d'injection (8) sont disposés sur l'élément de base (1) de manière à pouvoir tourner autour d'un axe d'injection commun.

9. Injecteur selon l'une des revendications précédentes,
**caractérisé en ce que**
l'élément de base (1) et/ou l'élément coulissant présentent au moins un chanfrein (1c, 1d) qui est disposé de telle manière que lors de l'opération d'injection, une rotation de l'élément coulissant par rapport à l'élément de base (1) soit effectuée dans une zone d'extrémité proximale lors du coulissement de l'élément coulissant dans la direction d'injection.

10. Injecteur selon les revendications 7 et 9,
**caractérisé en ce que**
l'élément coulissant (2) et l'élément de maintien de ressort d'éjection (8) présentent des surfaces d'appui correspondantes qui sont disposées de telle manière que l'élément d'éjection puisse être libéré d'une position de fixation par la rotation de l'élément coulissant, en particulier l'élément de maintien de ressort d'éjection (8) présente un prolongement et l'élément coulissant (2) présente une surface de guidage (2a) correspondante.

11. Injecteur selon l'une des revendications 9 à 10,
**caractérisé en ce que**
l'élément de base (1) et l'élément coulissant présentent des éléments de guidage correspondants qui sont réalisés et disposés de telle manière que l'élément coulissant (2) puisse être pivoté par rapport à l'élément de base (1) seulement dans la zone d'extrémité proximale.

12. Injecteur selon la revendication 4 et 9,
**caractérisé en ce que**
l'élément coulissant présente des fentes de guidage pour le prolongement de la partie supérieure de canule qui sont disposées de telle manière que la partie supérieure de canule puisse être coulissée à l'opposé de la direction d'injection après la rotation de l'élément coulissant dans la zone d'extrémité proximale.

13. Injecteur selon la revendication 12,
**caractérisé en ce que**
le chanfrein (1c, 1d) est réalisé sur l'élément coulissant en une extrémité proximale des fentes de guidage.

14. Injecteur selon l'une des revendications précédentes,
**caractérisé en ce que**
l'injecteur présente un ressort de contre-force (7a), lequel est disposé de manière à agir à l'encontre d'un coulissement de l'élément coulissant dans la direction d'injection entre l'élément de base (1) et l'élément coulissant.
